# FASCICULE DE BREVET EUROPEEN

(11) **EP 3 207 010 B1**
(45) Date de publication et mention de la délivrance du brevet: **19.05.2021**
(21) Numéro de dépôt: 15798153.1
(22) Date de dépôt: 07.10.2015
(51) Int. Cl.: C07C 17/20, C07C 17/25, C07C 19/01, C07C 21/18

(54) **COMPOSITIONS A BASE DE 1,1,1,2,3-PENTACHLOROPROPANE**
ZUSAMMENSETZUNG MIT 1,1,1,2,3-PENTACHLROPROPAN
COMPOSITIONS COMPRISING 1,1,1,2,3 PENTACHLOROPROPANE

(30) Priorité: 16.10.2014 FR 1459926
(43) Date de publication de la demande: 23.08.2017
(73) Titulaire: Arkema France, 92700 Colombes (FR)
(72) Inventeur: DEUR-BERT, Dominique, 69390 Charly (FR); WENDLINGER, Laurent, 69510 Soucieu en Jarrest (FR); COLLIER, Bertrand, 69230 Saint-Genis-Laval (FR)
(74) Mandataire: Arkema Patent
(86) Numéro de dépôt international: PCT/FR2015/052692
(87) Numéro de publication internationale: WO 2016/059322

(56) Documents cités:
- WO-A1-2013/088195
- US-A1- 2012 157 723
- US-A1- 2014 206 911

## Description

### DOMAINE DE L'INVENTION

La présente invention concerne des compositions à base de F-240db (1,1,1,2,3-pentachloropropane) ainsi que leur utilisation notamment pour la production de F-1234yf (2,3,3,3-tétrafluoropropène).

### ARRIERE-PLAN TECHNIQUE

Le F-1234yf est un composé d'intérêt majeur pour les systèmes de réfrigération et de climatisation, compte tenu des nouvelles réglementations environnementales.

Il est connu de produire les hydrofluorooléfines telles que le F-1234yf par fluoration d'hydrochlorooléfines ou d'hydrochlorocarbures notamment. Cette fluoration est généralement une fluoration catalytique utilisant l'acide fluorhydrique comme agent fluorant.

Parmi les voies permettant d'obtenir le F-1234yf, il est en particulier connu d'utiliser le F-240db (1,1,1,2,3-pentachloropropane) en tant que composé de départ. Il est fait référence par exemple au document WO 2013/088195 à cet égard. On connait également de US 2012/157723 un procédé de fabrication d'un hydrocarbure chloré par l'obtention d'un composé saturé via une étape d'addition de CCl4 sur un composé insaturé. On connait également par US 2014/206911 un procédé de préparation d'hydrocarbures chlorés à partir de tétrachloropropanes en présence d'un composé antimoine polyvalent.

Il est souhaitable de parvenir à produire du F-1234yf présentant une teneur faible en impuretés. En particulier, la formation de certaines impuretés toxiques et/ou inflammables et/ou qui sont difficiles à séparer du F-1234yf doit être minimisée.

Il existe donc un besoin de fournir des moyens permettant d'obtenir des compositions de F-1234yf de pureté satisfaisante.

### RESUME DE L'INVENTION

L'invention concerne en premier lieu une composition comprenant au moins 99 % en poids de 1,1,1,2,3-pentachloropropane, et comprenant au moins un composé choisi parmi une liste de composés supplémentaires consistant en les trichloropropanes, les tétrachloropropanes, les pentachloropropanes différents du 1,1,1,2,3-pentachloropropane, les hexachloropropanes, les heptachloropropanes, les dichloropropènes, les trichloropropènes, les tétrachloropropènes, les pentachloropropènes et l'hexachloropropène, ledit composé étant présent dans la composition en une teneur pondérale inférieure ou égale à 500 ppm.

Selon un mode de réalisation, ledit composé est présent dans la composition en une teneur pondérale inférieure ou égale à 250 ppm ; de préférence inférieure ou égale à 150 ppm ; plus particulièrement inférieure ou égale à 100 ppm; plus particulièrement inférieure ou égale à 50 ppm ; et idéalement inférieure ou égale à 10 ppm.

Selon un mode de réalisation, la composition comprend une pluralité de composés choisis parmi ladite liste de composés supplémentaires, chacun des composés de ladite pluralité de composés étant présent dans la composition en une teneur pondérale inférieure ou égale à 500 ppm ; de préférence inférieure ou égale à 250 ppm ; de préférence inférieure ou égale à 150 ppm; plus particulièrement inférieure ou égale à 100 ppm ; plus particulièrement inférieure ou égale à 50 ppm ; et idéalement inférieure ou égale à 10 ppm.

Selon un mode de réalisation, la composition comprend une pluralité de composés choisis parmi ladite liste de composés supplémentaires, la teneur pondérale totale de l'ensemble des composés de ladite liste étant inférieure ou égale à 1000 ppm; de préférence inférieure ou égale à 500 ppm ; de préférence inférieure ou égale à 250 ppm ; de préférence inférieure ou égale à 150 ppm; plus particulièrement inférieure ou égale à 100 ppm; plus particulièrement inférieure ou égale à 50 ppm ; et idéalement inférieure ou égale à 10 ppm.

Selon un mode de réalisation, la composition comprend au moins 99,5 % en poids, et de préférence au moins 99,8 % en poids, et de manière plus particulièrement préférée au moins 99,9 % en poids, de 1,1,1,2,3-pentachloropropane.

Selon un mode de réalisation, la composition comprend au moins un composé choisi parmi le groupe constitué du 1,1,1,3-tétrachloropropane, du 3,3,3-trichloropropène et du 1,1,3-trichloropropène ; et la teneur pondérale de chacun de ces composés dans la composition est inférieure ou égale à 100 ppm, de préférence inférieure ou égale à 50 ppm ; et, optionnellement, la teneur pondérale totale des composés de ce groupe dans la composition est inférieure ou égale à 100 ppm, de préférence inférieure ou égale à 50 ppm.

Selon un mode de réalisation, la composition comprend au moins un composé choisi parmi les trichloropropènes et les tétrachloropropanes, la teneur pondérale de chacun de ces composés dans la composition étant inférieure ou égale à 250 ppm, de préférence inférieure ou égale à 150 ppm ; et, optionnellement, la teneur pondérale totale des trichloropropènes et tétrachloropropanes dans la composition est inférieure ou égale à 250 ppm, de préférence inférieure ou égale à 150 ppm.

Selon un mode de réalisation, la composition comprend au moins un composé choisi parmi les pentachloropropènes et les hexachloropropanes, la teneur pondérale de chacun de ces composés dans la composition étant inférieure ou égale à 50 ppm, de préférence inférieure ou égale à 10 ppm ; et, optionnellement, la teneur pondérale totale des pentachloropropènes et hexachloropropanes dans la composition est inférieure ou égale à 50 ppm, de préférence inférieure ou égale à 10 ppm.

Selon un mode de réalisation, la composition comprend au moins un composé choisi parmi l'hexachloropropène et les heptachloropropanes, la teneur pondérale de chacun de ces composés dans la composition étant inférieure ou égale à 50 ppm, de préférence inférieure ou égale à 10 ppm ; et, optionnellement, la teneur pondérale totale de l'hexachloropropène et des heptachloropropanes dans la composition est inférieure ou égale à 50 ppm, de préférence inférieure ou égale à 10 ppm.

Selon un mode de réalisation, la composition comprend au moins un composé choisi parmi les dichloropropènes et les trichloropropanes, la teneur pondérale de chacun de ces composés dans la composition étant inférieure ou égale à 50 ppm, de préférence inférieure ou égale à 10 ppm; et, optionnellement, la teneur pondérale totale des dichloropropènes et trichloropropanes dans la composition est inférieure ou égale à 50 ppm, de préférence inférieure ou égale à 10 ppm.

Selon un mode de réalisation, la composition comprend au moins un composé choisi parmi le groupe constitué du 1,1,3,3-tétrachloropropène, du 1,3,3,3-tétrachloropropène, du 1,1,1,3,3-pentachloropropane et du 1,1,2,3,3-pentachloropropane, et la teneur pondérale de chacun de ces composés dans la composition est inférieure ou égale à 500 ppm, de préférence inférieure ou égale à 300 ppm ; et, optionnellement, la teneur pondérale totale des composés de ce groupe dans la composition est inférieure ou égale à 500 ppm, de préférence inférieure ou égale à 300 ppm.

L'invention concerne également un procédé de production de 2,3,3,3-tétrafluoropropène, comprenant :
- la fourniture d'une composition telle que décrite ci-dessus ;
- la réaction de cette composition avec de l'acide fluorhydrique en phase gazeuse.

Selon un mode de réalisation, le procédé comprend une unique étape de fluoration catalytique.

Selon un mode de réalisation, le procédé comprend deux étapes successives de fluoration catalytique, à savoir :
- la réaction de la composition avec de l'acide fluorhydrique en phase gazeuse, pour fabriquer un produit intermédiaire ;
- optionnellement, une purification du produit intermédiaire ; puis
- la réaction du produit intermédiaire avec de l'acide fluorhydrique en phase gazeuse, pour fabriquer le 2,3,3,3-tétrafluoropropène ;
le produit intermédiaire étant de préférence le 2-chloro-3,3,3-trifluoropropène.

La présente invention permet de surmonter les inconvénients de l'état de la technique. Elle fournit plus particulièrement des compositions à base de F-240db dont la teneur en impuretés permet de minimiser la présence d'impuretés néfastes dans le F-1234yf fabriqué à partir de celles-ci.

En effet, les impuretés présentes dans le F-1234yf dépendent pour partie des impuretés initialement présentes dans le F-240db qui est utilisé pour le fabriquer. Au cours de la ou des réactions de fluoration, certaines des impuretés du F-240db peuvent être converties en impuretés différentes dans le F-1234yf. La maitrise des impuretés présentes dans le F-240db permet donc indirectement de contrôler les impuretés présentes dans le F-1234yf.

Un tel contrôle indirect peut être avantageux dans la mesure où les impuretés du F-1234yf peuvent être plus difficiles à séparer du F-1234yf que les impuretés du F-240db par rapport au F-240db. C'est notamment le cas lorsque les impuretés du F-1234yf ont un point d'ébullition très proche ou forment un azéotrope ou un quasi-azéotrope avec celui-ci.

### DESCRIPTION DE MODES DE REALISATION DE L'INVENTION

L'invention est maintenant décrite plus en détail et de façon non limitative dans la description qui suit.

Toutes les teneurs indiquées sont des teneurs pondérales sauf mention contraire.

### Nomenclature

Le tableau suivant fournit la nomenclature d'un certain nombre de composés qui interviennent dans l'invention.

| **Formule** | **Notation** | **Nom complet** |
|---|---|---|
| CF₃-CH₃ | F-143a | 1,1,1-trifluoroéthane |
| CCl₃-CHCl-CCl₃ | F-220da | 1,1,1,2,3,3,3-heptachloropropane |
| CHCl₂-CCl₂-CCl₃ | F-220aa | 1,1,1,2,2,3,3-heptachloropropane |
| CF₃-CHCl-CF₃ | F-226da | 2-chloro-1,1,1,3,3,3-hexafluoropropane |
| CF₃-CHF-CClF₂ | F-226ea | 1-chloro-1,1,2,3,3,3-hexafluoropropane |
| CF₃-CFCl-CHF₂ | F-226ba | 2-chloro-1,1,2,3,3,3-hexafluoropropane |
| CF₃-CF₂-CHFCl | F-226ca | 3-chloro-1,1,1,2,2,3-hexafluoropropane |
| CClF₂-CF₂-CHF₂ | F-226cb | 1-chloro-1,1,2,2,3,3-hexafluoropropane |
| CCl₃-CH₂-CCl₃ | F-230fa | 1,1,1,3,3,3-hexachloropropane |
| CHCl₂-CHCl-CCl₃ | F-230da | 1,1,1,2,3,3-hexachloropropane |
| CHCl₂-CCl₂-CHCl₂ | F-230aa | 1,1,2,2,3,3-hexachloropropane |
| CH₂Cl-CCl₂-CCl₃ | F-230ab | 1,1,1,2,2,3-hexachloropropane |
| CF₃-CH₂-CF₃Cl | F-235fa | 3-chloro-1,1,1,3,3-pentafluoropropane |
| CF₃-CHF-CHFCl | F-235ea | 1-chloro-1,2,3,3,3-pentafluoropropane |
| CHF₂-CHF-CClF₂ | F-235eb | 1-chloro-1,1,2,3,3-pentafluoropropane |
| CHClF-CF₂-CHF₂ | F-235ca | 3-chloro-1,1,2,2,3-pentafluoropropane |
| CH₂Cl-CF₂-CF₃ | F-235cb | 3-chloro-1,1,1,2,2-pentafluoropropane |
| CH₂F-CF₂-CClF₂ | F-235cc | 1-chloro-1,1,2,2,3-pentafluoropropane |
| CHF₂-CHCl-CF₃ | F-235da | 2-chloro-1,1,1,3,3-pentafluoropropane |
| CHF₂-CClF-CHF₂ | F-235ba | 2-chloro-1,1,2,3,3-pentafluoropropane |
| CH₂F-CClF-CF₃ | F-235bb | 2-chloro-1,1,1,2,3-pentafluoropropane |
| CF₃-C-CF₃ | F-236fa | 1,1,1,3,3,3-hexafluoropropane |
| CHF₂-CF₂-CHF₂ | F-236ca | 1,1,2,2,3,3-hexafluoropropane |
| CH₂F-CF₂-CF₃ | F-236cb | 1,1,1,2,2,3-hexafluoropropane |
| CHF₂-CHF-CF₃ | F-236ea | 1,1,1,2,3,3-hexafluoropropane |
| CHCl₂-CH₂-CCl₃ | F-240fa | 1,1,1,3,3-pentachloropropane |
| CHCl₂-CHCl-CHCl₂ | F-240da | 1,1,2,3,3-pentachloropropane |
| CH₂Cl-CHCl-CCl₃ | F-240db | 1,1,1,2,3-pentachloropropane |
| CH₂Cl-CCl₂-CHCl₂ | F-240aa | 1,1,2,2,3-pentachloropropane |
| CH₃-CCl₂-CCl₃ | F-240ab | 1,1,1,2,2-pentachloropropane |
| CH₂F-CF₂-CHF₂ | F-245ca | 1,1,2,2,3-pentafluoropropane |
| CF₃-CF₂-CH₃ | F-245cb | 1,1,1,2,2-pentafluoropropane |
| CHF₂-CHF-CHF₂ | F-245ea | 1,1,2,3,3-pentafluoropropane |
| CH₂F-CHF-CF₃ | F-245eb | 1,1,1,2,3-pentafluoropropane |
| CHF₂-CH₂-CF₃ | F-245fa | 1,1,1,3,3-pentafluoropropane |
| CHCl₂-CH₂-CHCl₂ | F-250fa | 1,1,3,3-tétrachloropropane |
| CH₂Cl-CH₂-CCl₃ | F-250fb | 1,1,1,3-tétrachloropropane |
| CH₂Cl-CHCl-CHCl₂ | F-250da | 1,1,2,3-tétrachloropropane |
| CH₃-CHCl-CCl₃ | F-250db | 1,1,1,2-tétrachloropropane |
| CH₂Cl-CCl₂-CH₂Cl | F-250aa | 1,2,2,3-tétrachloropropane |
| CH₃-CCl₂-CHCl₂ | F-250ab | 1,1,2,2-tétrachloropropane |
| CF₂Cl-CH₂-CH₂F | F-253fa | 1-chloro-1,1,3-trifluoropropane |
| CH₂Cl-CH₂-CF₃ | F-253fb | 1-chloro-3,3,3-trifluoropropane |
| CF₂Cl-CH₂-CH₂F | F-253fc | 1-chloro-1,1,3-trifluoropropane |
| CH₂F-CClF-CH₂F | F-253ba | 2-chloro-1,2,3-trifluoropropane |
| CHF₂-CClF-CH₃ | F-253bb | 2-chloro-1,1,2-trifluoropropane |
| CH₂Cl-CF₂-CH₂F | F-253ca | 1-chloro-2,2,3-trifluoropropane |
| CHFCl-CF₂-CH₃ | F-253cb | 1-chloro-1,2,2-trifluoropropane |
| CHF₂-CHF-CH₂Cl | F-253ea | 3-chloro-1,1,2-trifluoropropane |
| CHClF-CHF-CH₂F | F-253eb | 1-chloro-1,2,3-trifluoropropane |
| CClF₂-CHF-CH₃ | F-253ec | 1-chloro-1,1,2-trifluoropropane |
| CH₂Cl-CH₂-CHCl₂ | F-260fa | 1,1,3-trichloropropane |
| CH₃-CH₂-CCl₃ | F-260fb | 1,1,1-trichloropropane |
| CH₂Cl-CHCl-CH₂Cl | F-260da | 1,2, 3-trichloropropane |
| CH₃-CHCl-CHCl₂ | F-260db | 1,1,2-trichloropropane |
| CH₃-CCl₂-CH₂Cl | F-260aa | 1,2,2-trichloropropane |
| CCl₃-CCl=CCl₂ | F-1210xa | hexachloropropène |
| CF₃-CCl=CCl₂ | F-1213xa | 1,1,2-trichloro-3,3,3-trifluoropropène |
| CF₂Cl-CCl=CFCl | F-1213xb | 1,2,3-trichloro-1,3,3-trifluoropropène |
| CFCl₂-CCl=CF₂ | F-1213xc | 2,3,3-trichloro-1,1,3-trifluoropropène |
| CCl₃-CF=CF₂ | F-1213yc | 3,3,3-trichloro-1,1,2-trifluoropropène |
| CFCl₂-CF=CFCl | F-1213yb | 1,3,3-trichloro-1,2,3-trifluoropropène |
| CF₂Cl-CF=CCl₂ | F-1213ya | 1,1,3-trichloro-2,3,3-trifluoropropène |
| CCl₂F-CF=CF₂ | F-1214yc | 3,3-dichloro-1,1,2,3-tétrafluoropropène |
| CClF₂-CCl=CF₂ | F-1214xc | 2,3-dichloro-1,1,3,3-tétrafluoropropène |
| CClF₂-CF=CFCl | F-1214yb | 1,3-dichloro-1,2,3,3-tétrafluoropropène |
| CF₃-CCl=CFCl | F-1214xb | 1,2-dichloro-1,3,3,3-tétrafluoropropène |
| CF₃-CF=CCl₂ | F-1214ya | 1,2-dichloro-2,3,3,3-tétrafluoropropène |
| CF₃-CF=CF₂ | F-1216yc | hexafluoropropène |
| CHCl₂-CCl=CCl₂ | F-1220xa | 1,1,2,3,3-pentachloropropène |
| CCl₃-CCl=CHCl | F-1220xd | 1,2,3,3,3-pentachloropropène |
| CCl₃-CH=CCl₂ | F-1220za | 1,1,3,3,3-pentachloropropène |
| CF₃-CCl=CHCl | F-1223xd | 1,2-dichloro-3,3,3-trifluoropropène |
| CF₂Cl-CCl=CHF | F-1223xe | 2,3-dichloro-1,3,3-trifluoropropène |
| CHFCl-CCl=CF₂ | F-1223xc | 2,3-dichloro-1,1,3-trifluoropropène |
| CFCl₂-CH=CF₂ | F-1223zc | 3,3-dichloro-1,1,3-trifluoropropène |
| CF₂Cl-CH=CFCl | F-1223zb | 1,3-dichloro-1,3,3-trifluoropropène |
| CF₃-CH=CCl₂ | F-1223za | 1,1-dichloro-3,3,3-trifluoropropène |
| CHF₂-CF=CCl₂ | F-1223ya | 1,1-dichloro-2,3,3-trifluoropropène |
| CF₂Cl-CF=CHCl | F-1223yd | 1,3-dichloro-2,3,3-trifluoropropène |
| CFCl₂-CF=CHF | F-1223ye | 3,3-dichloro-1,2,3-trifluoropropène |
| CHCl₂-CF=CF₂ | F-1223yc | 3,3-dichloro-1,1,2-trifluoropropène |
| CHFCl-CF=CF₂ | F-1224yc | 3-chloro-1,1,2,3-tétrafluoropropène |
| CHF₂-CCl=CF₂ | F-1224xc | 2-chloro-1,1,3,3-tétrafluoropropène |
| CF₂Cl-CH=CF₂ | F-1224zc | 3-chloro-1,1,3,3-tétrafluoropropène |
| CHF₂-CF=CFCl | F-1224yb | 1-chloro-1,2,3,3-tétrafluoropropène |
| CF₃-CH=CFCl | F-1224zb | 1-chloro-1,3,3,3-tétrafluoropropène |
| CClF₂-CF=CHF | F-1224ye | 3-chloro-1,2,3,3-tétrafluoropropène |
| CF₃-CCl=CHF | F-1224xe | 2-chloro-1,3,3,3-tétrafluoropropène |
| CF₃-CF=CHCl | F-1224yd | 1-chloro-2,3,3,3-tétrafluoropropène |
| CF₃-CH=CF₂ | F-1225zc | 1,1,3,3,3-pentafluoropropène |
| CHF₂-CF=CF₂ | F-1225yc | 1,1,2,3,3-pentafluoropropène |
| CF₃-CF=CHF | F-1225ye | 1,2,3,3,3-pentafluoropropène |
| CH₂Cl-CCl=CCl₂ | F-1230xa | 1,1,2,3-tétrachloropropène |
| CHCl₂-CCl=CHCl | F-1230xd | 1,2,3,3-tétrachloropropène |
| CCl₃-CCl=CH₂ | F-1230xf | 2,3,3,3-tétrachloropropène |
| CHCl₂-CH=CCl₂ | F-1230za | 1,1,3,3-tétrachloropropène |
| CCl₃-CH=CHCl | F-1230zd | 1,3,3,3-tétrachloropropène |
| CF₃-CCl=CH₂ | F-1233xf | 2-chloro-3,3,3-trifluoropropène |
| CClF₂-CF=CH₂ | F-1233yf | 3-chloro-2,3,3-trifluoropropène |
| CHF₂-CF=CHCl | F-1233yd | 1-chloro-2,3,3-trifluoropropène |
| CF₃-CH=CHCl | F-1233zd | 1-chloro-3,3,3-trifluoropropène |
| CHF₂-CCl=CHF | F-1233xe | 2-chloro-1,3,3-trifluoropropène |
| CHClF-CF=CHF | F-1233ye | 3-chloro-1,2,3-trifluoropropène |
| CClF₂-CH=CHF | F-1233ze | 3-chloro-1,3,3-trifluoropropène |
| CH₂Cl-CF=CF₂ | F-1233yc | 3-chloro-1,1,2-trifluoropropène |
| CFH₂-CCl=CF₂ | F-1233xc | 2-chloro-1,1,3-trifluoropropène |
| CFClH-CH=CF₂ | F-1233zc | 3-chloro-1,1,3-trifluoropropène |
| CFH₂-CF=CFCl | F-1233yb | 1-chloro-1,2,3-trifluoropropène |
| CF₂H-CH=CFCl | F-1233zb | 1-chloro-1,3,3-trifluoropropène |
| CF₃-CF=CH₂ | F-1234yf | 2,3,3,3-tétrafluoropropène |
| CF₃-CH=CHF | F-1234ze | 1,3,3,3-tétrafluoropropène |
| CH₂F-CF=CF₂ | F-1234yc | 1,1,2,3-tétrafluoropropène |
| CHF₂-CH=CF₂ | F-1234zc | 1,1,3,3-tétrafluoropropène |
| CHF₂-CF=CHF | F-1234ye | 1,2,3,3-tétrafluoropropène |
| CH₃-CCl=CCl₂ | F-1240xa | 1,1,2-trichloropropène |
| CH₂Cl-CCl=CHCl | F-1240xd | 1,2,3-trichloropropène |
| CHCl₂-CCl=CH₂ | F-1240xf | 2,3,3-trichloropropène |
| CH₂Cl-CH=CCl₂ | F-1240za | 1,1,3-trichloropropène |
| CHCl₂-CH=CHCl | F-1240zd | 1,3,3-trichloropropène |
| CCl₃-CH=CH₂ | F-1240zf | 3,3,3-trichloropropène |
| CClF₂-CH=CH₂ | F-1242zf | 3-chloro-3,3-difluoropropène |
| CHClF-CF=CH₂ | F-1242yf | 3-chloro-2,3-difluoropropène |
| CHF₂-CCl=CH₂ | F-1242xf | 2-chloro-3,3-difluoropropène |
| CH₃-CCl=CF₂ | F-1242xc | 2-chloro-1,1-difluoropropène |
| CH₂Cl-CH=CF₂ | F-1242zc | 3-chloro-1,1-difluoropropène |
| CH₂Cl-CF=CHF | F-1242ye | 3-chloro-1,2-difluoropropène |
| CH₂F-CCl=CHF | F-1242xe | 2-chloro-1,3-difluoropropène |
| CHFCl-CH=CHF | F-1242ze | 3-chloro-1,3-difluoropropène |
| CH₂F-CF=CHCl | F-1242yd | 1-chloro-2,3-difluoropropène |
| CHF₂-CH=CHCl | F-1242zd | 1-chloro-3,3-difluoropropène |
| CH₂F-CH=CF₂ | F-1243zc | 1,1,3-trifluoropropène |
| CH₃-CF=CF₂ | F-1243yc | 1,1,2-trifluoropropène |
| CF₃-CH=CH₂ | F-1243zf | 3,3,3-trifluoropropène |
| CH₂F-CF=CHF | F-1243ye | 1,2,3-trifluoropropène |
| CHF₂-CF=CH₂ | F-1243yf | 2,3,3-trifluoropropène |
| CHF₂-CH=CHF | F-1243ze | 1,3,3-trifluoropropène |
| CH₃-CH=CCl₂ | F-1250za | 1,1-dichloropropène |
| CH₃-CCl=CHCl | F-1250xd | 1,2-dichloropropène |
| CH₂Cl-CCl=CH₂ | F-1250xf | 2,3-dichloropropène |
| CH₂Cl-CH=CHCl | F-1250zd | 1,3-dichloropropène |
| CHCl₂-CH=CH₂ | F-1250zf | 3,3-dichloropropène |
| CH₃-CH=CF₂ | F-1252zc | 1,1-difluoropropène |
| CH₃-CF=CHF | F-1252ye | 1,2-difluoropropène |
| CH₂F-CF=CH₂ | F-1252yf | 2,3-difluoropropène |
| CHF₂-CH=CH₂ | F-1252zf | 3,3-difluoropropène |

Lorsque les composés ci-dessus existent sous la forme de deux isomères cis et trans, le nom du composé (par exemple le F-1234ze) désigne indifféremment l'une ou l'autre forme ou un mélange des deux formes. Les teneurs maximales indiquées sont alors des teneurs totales vis-à-vis des deux formes possibles.

Par ailleurs, on désigne de manière générique par F-220 l'ensemble des composés heptachloropropanes, par F-230 l'ensemble des composés hexachloropropanes et ainsi de suite, en utilisant les notations du tableau ci-dessus sans les deux lettres finales.

### Compositions selon l'invention

L'invention propose des compositions à base de F-240db. La teneur en F-240db est supérieure ou égale à 99 %.

Selon certains modes de réalisation, elle est supérieure ou égale à 99,1 %, ou à 99,2 %, ou à 99,3 %, ou à 99,4 %, ou à 99,5 %, ou à 99,6 %, ou à 99,7 %, ou à 99,8 %, ou à 99,9 %.

Les compositions selon l'invention comprennent également au moins un composé choisi parmi une liste de composés supplémentaires qui est constituée par les séries F-220, F-230, F-240 (à l'exception du F-240db), F-250, F-260 ainsi que par les séries F-1210, F-1220, F-1230, F-1240 et F-1250, ledit composé étant présent dans la composition en une teneur inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm; ou inférieure ou égale à 5 ppm.

Ledit au moins un composé peut être présent en une teneur supérieure ou égale à 1 ppm, ou supérieure ou égale à 2 ppm, ou supérieure ou égale à 3 ppm, ou supérieure ou égale à 5 ppm.

Par exemple, ledit au moins un composé peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Un mode de réalisation concerne de telles compositions qui comprennent une pluralité (deux, trois, quatre ou plus de quatre) composés choisi parmi la liste de composés supplémentaires ci-dessus, la teneur de chacun desdits composés étant inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm.

Chaque composé de cette pluralité peut alors être présent en une teneur supérieure ou égale à 1 ppm, ou supérieure ou égale à 2 ppm, ou supérieure ou égale à 3 ppm, ou supérieure ou égale à 5 ppm.

Par exemple, chaque composé de cette pluralité peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Un mode de réalisation concerne de telles compositions dans lesquelles la teneur de chacun des composés de la liste de composés supplémentaires ci-dessus éventuellement présent dans la composition (à l'exception du F-240aa, du F-1230xf et du F-1230xa, qui peuvent éventuellement être présents en quantités supérieures) est inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm.

Chaque composé de la liste de composés supplémentaires peut alors être présent en une teneur supérieure ou égale à 1 ppm, ou supérieure ou égale à 2 ppm, ou supérieure ou égale à 3 ppm, ou supérieure ou égale à 5 ppm.

Par exemple, chaque composé de la liste de composés supplémentaires (à l'exception du F-240aa, du F-1230xf et du F-1230xa, qui peuvent éventuellement être présents en quantités supérieures) peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Les compositions selon l'invention peuvent notamment comprendre un ou plusieurs composés de la série F-220, chacun étant présent dans la composition en une teneur inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm ; et la teneur totale en composés de la série F-220 dans la composition, de préférence, étant inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm; ou inférieure ou égale à 5 ppm.

Il est à noter que le ou les composés de la série F-220 peut alors être présent en une teneur supérieure ou égale à 1 ppm, ou supérieure ou égale à 2 ppm, ou supérieure ou égale à 3 ppm, ou supérieure ou égale à 5 ppm. De même, la teneur totale en composés de la série F-220 dans la composition peut être supérieure ou égale à 1 ppm, ou supérieure ou égale à 2 ppm, ou supérieure ou égale à 3 ppm, ou supérieure ou égale à 5 ppm.

Par exemple, le ou les composés de la série F-220 peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Par exemple, la teneur totale en composés de la série F-220 dans la composition peut être de 1 à 5 ppm ; ou de 5 à 10 ppm ; ou de 10 à 25 ppm ; ou de 25 à 50 ppm ; ou de 50 à 75 ppm ; ou de 75 à 100 ppm ; ou de 100 à 150 ppm ; ou de 150 à 200 ppm ; ou de 200 à 250 ppm ; ou de 250 à 300 ppm ; ou de 300 à 350 ppm ; ou de 350 à 400 ppm ; ou de 400 à 450 ppm ; ou de 450 à 500 ppm.

Les compositions selon l'invention peuvent notamment comprendre un ou plusieurs composés de la série F-230, chacun étant présent dans la composition en une teneur inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm ; et la teneur totale en composés de la série F-230 dans la composition, de préférence, étant inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm; ou inférieure ou égale à 5 ppm.

Il est à noter que le ou les composés de la série F-230 peut alors être présent en une teneur supérieure ou égale à 1 ppm, ou supérieure ou égale à 2 ppm, ou supérieure ou égale à 3 ppm, ou supérieure ou égale à 5 ppm. De même, la teneur totale en composés de la série F-230 dans la composition peut être supérieure ou égale à 1 ppm, ou supérieure ou égale à 2 ppm, ou supérieure ou égale à 3 ppm, ou supérieure ou égale à 5 ppm.

Par exemple, le ou les composés de la série F-230 peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Par exemple, la teneur totale en composés de la série F-230 dans la composition peut être de 1 à 5 ppm ; ou de 5 à 10 ppm ; ou de 10 à 25 ppm ; ou de 25 à 50 ppm ; ou de 50 à 75 ppm ; ou de 75 à 100 ppm ; ou de 100 à 150 ppm ; ou de 150 à 200 ppm ; ou de 200 à 250 ppm ; ou de 250 à 300 ppm ; ou de 300 à 350 ppm ; ou de 350 à 400 ppm ; ou de 400 à 450 ppm ; ou de 450 à 500 ppm.

Les compositions selon l'invention peuvent notamment comprendre un ou plusieurs composés de la série F-240, chacun (sauf le F-240db et sauf le F-240aa, qui peut également conduire au F-1234yf) étant présent dans la composition en une teneur inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm ; et la teneur totale en composés de la série F-240 (sauf F-240db et F-240aa) dans la composition, de préférence, étant inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm.

Il est à noter que le ou les composés de la série F-240 (sauf F-240db et F-240aa) peut alors être présent en une teneur supérieure ou égale à 1 ppm, ou supérieure ou égale à 2 ppm, ou supérieure ou égale à 3 ppm, ou supérieure ou égale à 5 ppm. De même, la teneur totale en composés de la série F-240 (sauf F-240db et F-240aa) dans la composition peut être supérieure ou égale à 1 ppm, ou supérieure ou égale à 2 ppm, ou supérieure ou égale à 3 ppm, ou supérieure ou égale à 5 ppm.

Par exemple, le ou les composés de la série F-240 (sauf F-240db et F-240aa) peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Par exemple, la teneur totale en composés de la série F-240 (sauf F-240db et F-240aa) dans la composition peut être de 1 à 5 ppm ; ou de 5 à 10 ppm ; ou de 10 à 25 ppm ; ou de 25 à 50 ppm ; ou de 50 à 75 ppm ; ou de 75 à 100 ppm ; ou de 100 à 150 ppm ; ou de 150 à 200 ppm ; ou de 200 à 250 ppm ; ou de 250 à 300 ppm ; ou de 300 à 350 ppm ; ou de 350 à 400 ppm ; ou de 400 à 450 ppm ; ou de 450 à 500 ppm.

Plus particulièrement, la teneur totale en F-240fa dans la composition peut être comprise entre 1 et 500 ppm, avantageusement entre 10 et 500 ppm, de préférence entre 50 et 450 ppm.

Le F-240db et le F-240aa peuvent être présents en quantités nettement supérieures à celles listées ci-dessus. Par exemple, la teneur totale en F-240aa peut être supérieure à 0,1%.

Les compositions selon l'invention peuvent notamment comprendre un ou plusieurs composés de la série F-250, chacun étant présent dans la composition en une teneur inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm ; et la teneur totale en composés de la série F-250 dans la composition, de préférence, étant inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm; ou inférieure ou égale à 5 ppm.

Il est à noter que le ou les composés de la série F-250 peut alors être présent en une teneur supérieure ou égale à 1 ppm, ou supérieure ou égale à 2 ppm, ou supérieure ou égale à 3 ppm, ou supérieure ou égale à 5 ppm. De même, la teneur totale en composés de la série F-250 dans la composition peut être supérieure ou égale à 1 ppm, ou supérieure ou égale à 2 ppm, ou supérieure ou égale à 3 ppm, ou supérieure ou égale à 5 ppm.

Par exemple, le ou les composés de la série F-250 peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Par exemple, la teneur totale en composés de la série F-250 dans la composition peut être de 1 à 5 ppm ; ou de 5 à 10 ppm ; ou de 10 à 25 ppm ; ou de 25 à 50 ppm ; ou de 50 à 75 ppm ; ou de 75 à 100 ppm ; ou de 100 à 150 ppm ; ou de 150 à 200 ppm ; ou de 200 à 250 ppm ; ou de 250 à 300 ppm ; ou de 300 à 350 ppm ; ou de 350 à 400 ppm ; ou de 400 à 450 ppm ; ou de 450 à 500 ppm.

Les compositions selon l'invention peuvent notamment comprendre un ou plusieurs composés de la série F-260, chacun étant présent dans la composition en une teneur inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm ; et la teneur totale en composés de la série F-260 dans la composition, de préférence, étant inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm; ou inférieure ou égale à 5 ppm.

Il est à noter que le ou les composés de la série F-260 peut alors être présent en une teneur supérieure ou égale à 1 ppm, ou supérieure ou égale à 2 ppm, ou supérieure ou égale à 3 ppm, ou supérieure ou égale à 5 ppm. De même, la teneur totale en composés de la série F-260 dans la composition peut être supérieure ou égale à 1 ppm, ou supérieure ou égale à 2 ppm, ou supérieure ou égale à 3 ppm, ou supérieure ou égale à 5 ppm.

Par exemple, le ou les composés de la série F-260 peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Par exemple, la teneur totale en composés de la série F-260 dans la composition peut être de 1 à 5 ppm ; ou de 5 à 10 ppm ; ou de 10 à 25 ppm ; ou de 25 à 50 ppm ; ou de 50 à 75 ppm ; ou de 75 à 100 ppm ; ou de 100 à 150 ppm ; ou de 150 à 200 ppm ; ou de 200 à 250 ppm ; ou de 250 à 300 ppm ; ou de 300 à 350 ppm ; ou de 350 à 400 ppm ; ou de 400 à 450 ppm ; ou de 450 à 500 ppm.

Les compositions selon l'invention peuvent notamment comprendre du F-1210xa, en une teneur inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm.

Il est à noter que le F-1210xa peut alors être présent en une teneur supérieure ou égale à 1 ppm, ou supérieure ou égale à 2 ppm, ou supérieure ou égale à 3 ppm, ou supérieure ou égale à 5 ppm.

Par exemple, le F-1210xa peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Les compositions selon l'invention peuvent notamment comprendre un ou plusieurs composés de la série F-1220, chacun étant présent dans la composition en une teneur inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm ; et la teneur totale en composés de la série F-1220 dans la composition, de préférence, étant inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm; ou inférieure ou égale à 5 ppm.

Il est à noter que le ou les composés de la série F-1220 peut alors être présent en une teneur supérieure ou égale à 1 ppm, ou supérieure ou égale à 2 ppm, ou supérieure ou égale à 3 ppm, ou supérieure ou égale à 5 ppm. De même, la teneur totale en composés de la série F-1220 dans la composition peut être supérieure ou égale à 1 ppm, ou supérieure ou égale à 2 ppm, ou supérieure ou égale à 3 ppm, ou supérieure ou égale à 5 ppm.

Par exemple, le ou les composés de la série F-1220 peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Par exemple, la teneur totale en composés de la série F-1220 dans la composition peut être de 1 à 5 ppm ; ou de 5 à 10 ppm ; ou de 10 à 25 ppm ; ou de 25 à 50 ppm ; ou de 50 à 75 ppm ; ou de 75 à 100 ppm ; ou de 100 à 150 ppm ; ou de 150 à 200 ppm ; ou de 200 à 250 ppm ; ou de 250 à 300 ppm ; ou de 300 à 350 ppm ; ou de 350 à 400 ppm ; ou de 400 à 450 ppm ; ou de 450 à 500 ppm.

Les compositions selon l'invention peuvent notamment comprendre un ou plusieurs composés de la série F-1230, chacun (sauf le F-1230xf et le F-1230xa, précurseurs du F-1234yf) étant présent dans la composition en une teneur inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm; ou inférieure ou égale à 5 ppm ; et la teneur totale en composés de la série F-1230 (à l'exception du F-1230xf et du F-1230xa) dans la composition, de préférence, étant inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm; ou inférieure ou égale à 5 ppm.

Il est à noter que le ou les composés de la série F-1230 (sauf F-1230xa et F-1230xf) peut alors être présent en une teneur supérieure ou égale à 1 ppm, ou supérieure ou égale à 2 ppm, ou supérieure ou égale à 3 ppm, ou supérieure ou égale à 5 ppm. De même, la teneur totale en composés de la série F-1230 (sauf F-1230xa et F-1230xf) dans la composition peut être supérieure ou égale à 1 ppm, ou supérieure ou égale à 2 ppm, ou supérieure ou égale à 3 ppm, ou supérieure ou égale à 5 ppm.

Par exemple, le ou les composés de la série F-1230 (sauf F-1230xa et F-1230xf) peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Par exemple, la teneur totale en composés de la série F-1230 (sauf F-1230xa et F-1230xf) dans la composition peut être de 1 à 5 ppm ; ou de 5 à 10 ppm ; ou de 10 à 25 ppm ; ou de 25 à 50 ppm ; ou de 50 à 75 ppm ; ou de 75 à 100 ppm ; ou de 100 à 150 ppm ; ou de 150 à 200 ppm ; ou de 200 à 250 ppm ; ou de 250 à 300 ppm ; ou de 300 à 350 ppm ; ou de 350 à 400 ppm ; ou de 400 à 450 ppm ; ou de 450 à 500 ppm.

Le F-1230xf et le F-1230xa peuvent être présents en quantités nettement supérieures à celles listées ci-dessus.

Les compositions selon l'invention peuvent notamment comprendre un ou plusieurs composés de la série F-1240, chacun étant présent dans la composition en une teneur inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm ; et la teneur totale en composés de la série F-1240 dans la composition, de préférence, étant inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm.

Il est à noter que le ou les composés de la série F-1240 peut alors être présent en une teneur supérieure ou égale à 1 ppm, ou supérieure ou égale à 2 ppm, ou supérieure ou égale à 3 ppm, ou supérieure ou égale à 5 ppm. De même, la teneur totale en composés de la série F-1240 dans la composition peut être supérieure ou égale à 1 ppm, ou supérieure ou égale à 2 ppm, ou supérieure ou égale à 3 ppm, ou supérieure ou égale à 5 ppm.

Par exemple, le ou les composés de la série F-1240 peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Par exemple, la teneur totale en composés de la série F-1240 dans la composition peut être de 1 à 5 ppm ; ou de 5 à 10 ppm ; ou de 10 à 25 ppm ; ou de 25 à 50 ppm ; ou de 50 à 75 ppm ; ou de 75 à 100 ppm ; ou de 100 à 150 ppm ; ou de 150 à 200 ppm ; ou de 200 à 250 ppm ; ou de 250 à 300 ppm ; ou de 300 à 350 ppm ; ou de 350 à 400 ppm ; ou de 400 à 450 ppm ; ou de 450 à 500 ppm.

Les compositions selon l'invention peuvent notamment comprendre un ou plusieurs composés de la série F-1250, chacun étant présent dans la composition en une teneur inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm ; et la teneur totale en composés de la série F-1250 dans la composition, de préférence, étant inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm.

Il est à noter que le ou les composés de la série F-1250 peut alors être présent en une teneur supérieure ou égale à 1 ppm, ou supérieure ou égale à 2 ppm, ou supérieure ou égale à 3 ppm, ou supérieure ou égale à 5 ppm. De même, la teneur totale en composés de la série F-1250 dans la composition peut être supérieure ou égale à 1 ppm, ou supérieure ou égale à 2 ppm, ou supérieure ou égale à 3 ppm, ou supérieure ou égale à 5 ppm.

Par exemple, le ou les composés de la série F-1250 peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Par exemple, la teneur totale en composés de la série F-1250 dans la composition peut être de 1 à 5 ppm ; ou de 5 à 10 ppm ; ou de 10 à 25 ppm ; ou de 25 à 50 ppm ; ou de 50 à 75 ppm ; ou de 75 à 100 ppm ; ou de 100 à 150 ppm ; ou de 150 à 200 ppm ; ou de 200 à 250 ppm ; ou de 250 à 300 ppm ; ou de 300 à 350 ppm ; ou de 350 à 400 ppm ; ou de 400 à 450 ppm ; ou de 450 à 500 ppm.

Le F-1243zf ayant induit une toxicité cardiaque chez le rat exposé de façon sub-chronique est une impureté particulièrement indésirable. Bien que le mécanisme d'action de cet effet et sa pertinence pour l'homme ne soit à ce jour pas établi, la teneur maximale admise en F-1243zf dans le F-1234yf pourrait être très faible. Or le F-1243zf forme un quasi-azéotrope avec le F-1234yf. Ces deux composés sont donc inséparables par distillation classique.

Par conséquent, il est souhaitable d'ajuster les compositions selon l'invention de façon à limiter la présence de précurseurs du F-1243zf dans celles-ci.

Des précurseurs possibles du F-1243zf (par réaction de fluoration) sont le F-1240za, le F-1240zf, le F-250fb (via l'un des deux précédents), le F-250da (via le F-1240za) et le F-250db (via le F-1240zf).

Ainsi, des compositions avantageuses selon l'invention :
- comprennent au moins un composé parmi ceux des séries F-1240 et F-250, dans une teneur : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent un ou plusieurs composés parmi ceux des séries F-1240 et F-250, la teneur totale de tous ces composés étant: inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent au moins un composé parmi le F-1240za, le F-1240zf, le F-250fb, le F-250da et le F-250db, dans une teneur : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent un ou plusieurs composés parmi le F-1240za, le F-1240zf, le F-250fb, le F-250da et le F-250db, la teneur totale de tous ces composés étant : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm.

Le F-1225ye a également montré un certain degré de toxicité chez le rat exposé de façon sub-chronique. Par ailleurs, c'est une substance inflammable. Il est donc souhaitable de limiter sa présence en mélange avec le F-1234yf (par exemple à une teneur inférieure ou égale à 5 ppm), et pour cela il est souhaitable de limiter la présence de ses précurseurs en mélange avec le F-240db.

Des précurseurs possibles du F-1225ye (par réaction de fluoration) sont le F-1220xd, le F-230da (via le F-1220xd) et le F-230ab (via le F-1220xd).

Ainsi, des compositions avantageuses selon l'invention :
- comprennent au moins un composé parmi ceux des séries F-1220 et F-230, dans une teneur : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent un ou plusieurs composés parmi ceux des séries F-1220 et F-230, la teneur totale de tous ces composés étant: inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent au moins un composé parmi le F-1220xd, le F-230da et le F-230ab, dans une teneur : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent un ou plusieurs composés parmi le F-1220xd, le F-230da et le F-230ab, la teneur totale de tous ces composés étant : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm.

Le F-1225zc est un autre composé présentant différents effets toxicologiques potentiels. Il est donc souhaitable de limiter sa présence dans le F-1234yf. Mais son point d'ébullition est proche de celui du F-1234yf, ce qui rend sa séparation par distillation classique difficile.

Par conséquent, il est souhaitable d'ajuster les compositions selon l'invention de façon à limiter la présence de précurseurs du F-1225zc dans celles-ci.

Des précurseurs possibles du F-1225zc (par réaction de fluoration) sont le F-1220za, le F-230fa (via le F-1220za) et le F-230da (via le F-1220za).

Ainsi, des compositions avantageuses selon l'invention :
- comprennent au moins un composé parmi le F-1220za, le F-230fa et le F-230da, dans une teneur : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent un ou plusieurs composés parmi le F-1220za, le F-230fa et le F-230da, la teneur totale de tous ces composés étant : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm.

Plus généralement, on pense que, du fait de leur réactivité, les molécules qui présentent un groupement =CF₂ ont également des risques d'effets toxicologiques Cela concerne, outre le F-1225zc, le F-1234yc, le F-1234zc, le F-1225yc, le F-1243zc, le F-1243yc, le F-1252zc et le F-1216yc. Parmi ces composés, les plus gênants sont le F-1216yc, le F-1243yc et le F-1252zc, en raison des difficultés à séparer ces composés du F-1234yf du fait de leur point d'ébullition proche de celui du F-1234yf.

Par conséquent, il est souhaitable d'ajuster les compositions selon l'invention de façon à limiter la présence de précurseurs du F-1216yc dans celles-ci.

Des précurseurs possibles du F-1216yc (par réaction de fluoration) sont le F-1210xa, le F-220da (via le F-1210xa) et le F-220aa (via le F-1210xa).

Ainsi, des compositions avantageuses selon l'invention :
- comprennent au moins un composé parmi ceux des séries F-1210 et F-220, dans une teneur : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent un ou plusieurs composés parmi ceux des séries F-1210 et F-220, la teneur totale de tous ces composés étant: inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent au moins un composé parmi le F-1210xa, le F-220da et le F-220aa, dans une teneur : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent un ou plusieurs composés parmi le F-1210xa, le F-220da et le F-220aa, la teneur totale de tous ces composés étant : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm.

Il est également souhaitable d'ajuster les compositions selon l'invention de façon à limiter la présence de précurseurs du F-1243yc dans celles-ci.

Des précurseurs possibles du F-1243yc (par réaction de fluoration) sont le F-1240xa et le F-250ab (via le F-1240xa).

Ainsi, des compositions avantageuses selon l'invention :
- comprennent au moins un composé parmi le F-1240xa et le F-250ab, dans une teneur: inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent un ou plusieurs composés parmi le F-1240xa et le F-250ab, la teneur totale de tous ces composés étant : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm.

Il est également souhaitable d'ajuster les compositions selon l'invention de façon à limiter la présence de précurseurs du F-1252zc dans celles-ci.

Des précurseurs possibles du F-1252zc (par réaction de fluoration) sont le F-1250za, le F-260fb (via le F-1250za) et le F-260db (via le F-1250za).

Ainsi, des compositions avantageuses selon l'invention :
- comprennent au moins un composé parmi ceux des séries F-1250 et F-260, dans une teneur : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent un ou plusieurs composés parmi ceux des séries F-1250 et F-260, la teneur totale de tous ces composés étant: inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent au moins un composé parmi le F-1250za, le F-260fb et le F-260db, dans une teneur : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent un ou plusieurs composés parmi le F-1250za, le F-260fb et le F-260db, la teneur totale de tous ces composés étant : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm.

Par ailleurs, le F-1234ze est une substance qui ne doit pas être présente en quantité trop élevée en mélange avec le F-1234yf. Par exemple, la teneur de F-1234ze dans le F-1234yf devrait rester inférieure ou égale à 500 ppm. Or le point d'ébullition du F-1234ze est proche de celui du F-1234yf, ce qui rend une séparation par distillation conventionnelle difficile. Pour cette raison, il est souhaitable de limiter la présence des précurseurs du F-1234ze en mélange avec le F-240db.

Des précurseurs possibles du F-1234ze (par réaction de fluoration) sont le F-1230za, le F-1230zd, le F-240fa et le F-240da.

Ainsi, des compositions avantageuses selon l'invention :
- comprennent au moins un composé parmi le F-1230za, le F-1230zd, le F-240fa et le F-240da, dans une teneur : inférieure ou égale à 500 ppm ; ou de 400 à 500 ppm ; ou de 300 à 400 ppm ; ou de 200 à 300 ppm ; ou de 100 à 200 ppm ; ou inférieure ou égale à 100 ppm, et par exemple de 1 à 100 ppm ; ou bien
- comprennent un ou plusieurs composés parmi le F-1230za, le F-1230zd, le F-240fa et le F-240da, la teneur totale de tous ces composés étant : inférieure ou égale à 500 ppm ; ou de 400 à 500 ppm ; ou de 300 à 400 ppm ; ou de 200 à 300 ppm ; ou de 100 à 200 ppm ; ou inférieure ou égale à 100 ppm, et par exemple de 1 à 100 ppm.

Compte tenu de ce qui précède, des compositions avantageuses selon l'invention :
- comprennent un ou plusieurs composés parmi le F-1240za, le F-1240zf, le F-250fb, le F-250da et le F-250db, la teneur totale de tous ces composés étant : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; et en outre
- comprennent un ou plusieurs composés parmi le F-1220za, le F-230fa et le F-230da, la teneur totale de tous ces composés étant : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; et en outre
- comprennent un ou plusieurs composés parmi le F-1210xa, le F-220da et le F-220aa, la teneur totale de tous ces composés étant : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; et en outre
- comprennent un ou plusieurs composés parmi le F-1240xa et le F-250ab, la teneur totale de tous ces composés étant : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; et en outre
- comprennent un ou plusieurs composés parmi le F-1250za, le F-260fb et le F-260db, la teneur totale de tous ces composés étant : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; et en outre
- comprennent un ou plusieurs composés parmi le F-1220xd, le F-230da et le F-230ab, la teneur totale de tous ces composés étant : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; et en outre
- comprennent un ou plusieurs composés parmi le F-1230za, le F-1230zd, le F-240fa et le F-240da, la teneur totale de tous ces composés étant : inférieure ou égale à 500 ppm ; ou de 400 à 500 ppm ; ou de 300 à 400 ppm ; ou de 200 à 300 ppm ; ou de 100 à 200 ppm ; ou inférieure ou égale à 100 ppm, et par exemple de 1 à 100 ppm.

En outre, le F-250fb, le F-1240za et le F-1240zf peuvent être des composés intermédiaires dans la production du F-240db, comme exposé ci-dessous. Ce sont par ailleurs des précurseurs du F-1243zf et du F-1243yc. Par conséquent, des compositions avantageuses selon l'invention :
- comprennent au moins un composé parmi le F-250fb, le F-1240za et le F-1240zf, dans une teneur : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent un ou plusieurs composés parmi le F-250fb, le F-1240za et le F-1240zf, la teneur totale de tous ces composés étant : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm.

### Préparation des compositions selon l'invention

La fabrication du F-240db est connue par exemple du document US 8,304,589, auquel il est fait expressément référence ici. Le document propose un procédé en trois étapes :
- réaction du tétrachlorure de carbone avec de l'éthylène pour produire du F-250fb ;
- déshydrochloration thermique du F-250fb pour obtenir le F-1240za ou le F-1240zf ; et
- addition de chlore au F-1240za ou F-1240zf pour obtenir le F-240db.

Certaines variantes de ce procédé sont également décrites dans les documents US 4,650,914, US 2009/0216055, US 2014/0206911 et US 2013/0165705, auxquels il est fait expressément référence ici.

Un procédé en deux étapes peut également être envisagé, comme décrit dans le document US 3,446,859, auquel il est fait expressément référence ici :
- réaction du chlorure de méthyle sur le tétrachloroéthylène pour produire du F-1230xa ;
- hydrochloration du F-1230xa pour obtenir le F-240db.

Un procédé en une étape peut également être envisagé par réaction du dichlorométhane sur le trichloroéthylène.

Les compositions selon l'invention peuvent ensuite être obtenues en procédant un à une ou plusieurs étapes de séparation du F-240db vis-à-vis des autres composés mentionnés ci-dessus.

Ces étapes de séparation peuvent de préférence être effectuées par absorption / lavage et distillation. Alternativement à la distillation classique ou en combinaison avec celle-ci, il est également possible de prévoir une séparation par distillation extractive, des séparations physico-chimiques sur tamis moléculaire, alumine ou charbon actif ou une séparation membranaire.

Une première séparation est généralement réalisée en utilisant une distillation (colonne à plateaux, colonne à garnissage) à pression atmosphérique ou sous pression réduite. La pression choisie est inférieure à 760 mmHg, préférentiellement inférieure à 450 mmHg et plus préférentiellement inférieure à 200 mmHg. De façon inhérente, la pression de la colonne détermine les conditions de température pour un degré de séparation choisi. Le F-240db peut être récupéré en opérant la distillation à une température inférieure à 180°C, préférentiellement inférieure à 160°C et plus préférentiellement inférieure à 130°C. Une simple colonne ou un train de distillation peut être utilisé. Dans des conditions choisies, la pureté du F-240db après distillation atteint au minimum 99,8 %.

Une seconde séparation peut être réalisée en utilisant une adsorption sur zéolite ou charbon actif.

Les zéolites ou charbons actifs utilisables dans le procédé de purification de F-240db présentent avantageusement une taille moyenne de pores de 3,4 à 11 Å, de préférence de 3,4 à 10 Å. Si la zéolite ou le charbon actif a une taille de pores moyenne supérieure à 11 Å, la quantité de F-240db adsorbée augmente, alors que si la taille moyenne des pores est inférieure à 3,4 Å, la capacité d'adsorption de la zéolite ou du charbon actif est réduite.

La zéolite a de préférence un rapport Si / Al de 2 ou moins. Si le rapport Si / Al de la zéolite est supérieur à deux, certaines impuretés sont susceptibles de ne pas être adsorbées sélectivement. La zéolite est de préférence au moins un élément choisi dans le groupe consistant en des tamis moléculaires 4A, un tamis moléculaire 5A, un tamis moléculaire 10X et des tamis moléculaires 13X. En utilisant ces zéolites, la teneur en eau dans le F-240db peut également être réduite en même temps.

La zéolite et le charbon actif sont de préférence utilisés individuellement en vue de la régénération de l'adsorbant, mais ceux-ci peuvent également être utilisés en mélange. Les proportions de zéolite et de charbon actif dans le mélange ne sont pas particulièrement importantes, toutefois, il est préférable d'utiliser une quantité de zéolite plus élevée, ce qui permet de réduire la teneur en eau dans le F-240db.

Pour traiter le F-240db avec la zéolite et / ou le charbon actif en phase liquide, on peut utiliser un procédé discontinu (ou « batch ») ou un procédé continu. Industriellement, un procédé consistant à faire passer en continu le F-240db sur un lit fixe est préférable. La vitesse spatiale horaire liquide (VSHL) peut être choisie de manière appropriée en fonction de la teneur en impuretés à éliminer et de la quantité de F-240db à traiter. En général, la vitesse spatiale est de préférence de 1 à 50 h⁻¹. Industriellement le procédé de purification peut utiliser alternativement deux tours d'adsorption.

La température de traitement du F-240db est de 0°C à 120°C, de préférence de 20°C à 80°C. Si la température de traitement est supérieure à 120°C, le coût de équipement peut augmenter en raison du chauffage de l'appareil, tandis que si la température de traitement est inférieure à 0°C un équipement de refroidissement peut être nécessaire. La pression est de 0 à 3 MPa, de préférence de 0 à 1 MPa. Si la pression est supérieure à 3 MPa, la rentabilité peut diminuer en raison des exigences en matière de résistance à la pression de l'appareil.

Une technique de séparation membranaire peut également être mise en oeuvre en complément d'une adsorption sur charbon actif ou sur zéolite, ou bien en alternative à ces techniques. La séparation membranaire peut être mise en oeuvre en phase gaz selon un procédé continu opéré à faible pression, ou à pression réduite. La pression choisie est inférieure à 5 bar, préférentiellement inférieure à 2 bar et plus préférentiellement inférieure à la pression atmosphérique. Le choix de la membrane dépend des propriétés des impuretés à séparer du F-240db (différence de solubilité, de diffusivité et de perméabilité). La séparation membranaire est réalisée à une température dépendant de la pression choisie, inférieure à 250°C, préférentiellement inférieure à 230°C et plus préférentiellement inférieure à 180°C.

Lorsque le F-240db contenant des impuretés est mis en contact avec la zéolite et / ou le charbon actif en phase liquide et / ou est purifié sur membrane en phase gazeuse dans les conditions décrites ci-dessus, le F-240db peut être obtenu avec une pureté supérieure à 99,9 %.

### Fabrication du F-1234vf

Les compositions selon l'invention peuvent être utilisées pour la fabrication de F-1234yf présentant des spécifications souhaitées, par une ou plusieurs étapes de fluoration.

La fluoration est de préférence une fluoration catalytique en phase gazeuse par du HF.

Le catalyseur utilisé peut être par exemple à base d'un métal comprenant un oxyde de métal de transition ou un dérivé ou un halogénure ou un oxyhalogénure d'un tel métal. On peut citer par exemple FeCl₃, l'oxyfluorure de chrome, les oxydes de chrome (éventuellement soumis à des traitements de fluoration), les fluorures de chrome et leurs mélanges. D'autres catalyseurs possibles sont les catalyseurs supportés sur du carbone, les catalyseurs à base d'antimoine, les catalyseurs à base d'aluminium (par exemple AlF₃ et Al₂O₃, l'oxyfluorure d'alumine et le fluorure d'alumine).

On peut utiliser en général un oxyfluorure de chrome, un fluorure ou un oxyfluorure d'aluminium, ou un catalyseur supporté ou non contenant un métal tel que Cr, Ni, Fe, Zn, Ti, V, Zr, Mo, Ge, Sn, Pb, Mg, Sb.

On peut faire référence à cet égard au document WO 2007/079431 (en p.7, I.1-5 et 28-32), au document EP 939071 (paragraphe [0022]), au document WO 2008/054781 (en p.9 I.22-p.10 I.34), et au document WO 2008/040969 (revendication 1), auxquels il est fait expressément référence.

Le catalyseur est de manière plus particulièrement préférée à base de chrome et il s'agit plus particulièrement d'un catalyseur mixte comprenant du chrome.

Selon un mode de réalisation, on utilise un catalyseur mixte comprenant du chrome et du nickel. Le rapport molaire Cr / Ni (sur la base de l'élément métallique) est généralement de 0,5 à 5, par exemple de 0,7 à 2, par exemple d'environ 1. Le catalyseur peut contenir de 0,5 à 20 % en poids de chrome, et de 0,5 à 20 % en poids de nickel, de préférence de 2 à 10 % de chaque.

Le métal peut être présent sous forme métallique ou sous forme de dérivé, par exemple un oxyde, halogénure ou oxyhalogénure. Ces dérivés sont de préférence obtenus par activation du métal catalytique.

Le support est de préférence constitué avec de l'aluminium, par exemple de l'alumine, de l'alumine activée ou des dérivés d'aluminium, tels que les halogénures d'aluminium et les oxyhalogénures d'aluminium, par exemple décrits dans le document US 4,902,838, ou obtenus par le procédé d'activation décrit ci-dessus.

Le catalyseur peut comprendre du chrome et du nickel sous une forme activée ou non, sur un support qui a été soumis à une activation ou non.

On peut se reporter au document WO 2009/118628 (notamment en p.4, I.30-p.7 I.16), auquel il est fait expressément référence ici.

Un autre mode de réalisation préféré repose sur un catalyseur mixte contenant du chrome et au moins un élément choisi parmi Mg et Zn. Le rapport atomique de Mg ou Zn/Cr est de préférence de 0,01 à 5.

Avant son utilisation, le catalyseur est de préférence soumis à une activation avec de l'air, de l'oxygène ou du chlore et/ou avec de l'HF.

Par exemple, le catalyseur est de préférence soumis à une activation avec de l'air ou de l'oxygène et du HF à une température de 100 à 500°C, de préférence de 250 à 500°C et plus particulièrement de 300 à 400°C. La durée d'activation est de préférence de 1 à 200 h et plus particulièrement de 1 à 50 h.

Cette activation peut être suivie d'une étape d'activation de fluoration finale en présence d'un agent d'oxydation, d'HF et de composés organiques.

Le rapport molaire HF / composés organiques est de préférence de 2 à 40 et le rapport molaire agent d'oxydation / composés organiques est de préférence de 0,04 à 25. La température de l'activation finale est de préférence de 300 à 400°C et sa durée de préférence de 6 à 100 h.

La réaction de fluoration en phase gazeuse peut être effectuée :
- avec un rapport molaire HF / composé chloré de 1:1 à 150:1, de préférence de 3:1 à 100:1 et de manière plus particulièrement préférée de 5:1 à 50:1 ;
- avec un temps de contact de 1 à 100 s, de préférence 1 à 50 s et plus particulièrement 2 à 40 s (volume de catalyseur divisé par le flux entrant total, ajusté à la température et à la pression de fonctionnement) ;
- à une pression absolue allant de 0,1 à 50 bar, de préférence de 0,3 à 15 bar ;
- à une température (température du lit de catalyseur) de 100 à 500°C, de préférence de 200 à 450°C, et plus particulièrement de 250 à 400°C.

La durée de l'étape de réaction est typiquement de 10 à 2000 heures, de préférence de 50 à 500 heures et de manière plus particulièrement préférée de 70 à 300 heures.

Un agent oxydant, de préférence l'oxygène, peut éventuellement être ajouté lors de la réaction de fluoration. Le rapport molaire oxygène / composés organiques peut être de 0,005 à 2, de préférence de 0,01 à 1,5. L'oxygène peut être introduit pur ou sous forme d'air ou de mélange oxygène / azote. On peut également remplacer l'oxygène par du chlore.

Le flux de produits issu de la fluoration peut subir des traitements appropriés (distillation, lavage, etc.) afin de récupérer le F-1234yf sous forme purifiée et le séparer des autres composés présents (HCl, HF non réagi, F-240db non réagi, autres composés organiques). Un ou plusieurs flux peuvent faire l'objet d'un recyclage.

On peut également prévoir des étapes de régénération du catalyseur, comme décrit par exemple dans les documents WO 2012/098421 et WO 2012/098422 auxquels il est fait expressément référence.

On peut également prévoir une production de F-1234yf en deux étapes : d'abord la fluoration de la composition à base de F-240db pour produire du F-1233xf, puis la fluoration de ce dernier pour produire le F-1234yf. On peut utiliser un même réacteur ou des réacteurs successifs pour mettre en œuvre ces étapes. Ce type de procédé est décrit notamment dans le document WO 2013/088195 auquel il est fait expressément référence.

Le flux de F-1234yf obtenu, de préférence, contient :
- moins de 500 ppm, ou 250 ppm, ou 200 ppm, ou 150 ppm, ou 100 ppm, ou 50 ppm, ou 25 ppm, ou 10 ppm, ou 5 ppm, de F-1243zf ; et/ou
- moins de 500 ppm, ou 250 ppm, ou 200 ppm, ou 150 ppm, ou 100 ppm, ou 50 ppm, ou 25 ppm, ou 10 ppm, ou 5 ppm, de F-1225zc ; et/ou
- moins de 500 ppm, ou 250 ppm, ou 200 ppm, ou 150 ppm, ou 100 ppm, ou 50 ppm, ou 25 ppm, ou 10 ppm, ou 5 ppm, de F-1216yc ; et/ou
- moins de 500 ppm, ou 250 ppm, ou 200 ppm, ou 150 ppm, ou 100 ppm, ou 50 ppm, ou 25 ppm, ou 10 ppm, ou 5 ppm, de F-1243yc ; et/ou
- moins de 500 ppm, ou 250 ppm, ou 200 ppm, ou 150 ppm, ou 100 ppm, ou 50 ppm, ou 25 ppm, ou 10 ppm, ou 5 ppm, de F-1252zc ; et/ou
- moins de 500 ppm, ou 250 ppm, ou 200 ppm, ou 150 ppm, ou 100 ppm, ou 50 ppm, ou 25 ppm, ou 10 ppm, ou 5 ppm, de F-1225ye ; et/ou
- moins de 500 ppm, ou 250 ppm, ou 200 ppm, ou 150 ppm, ou 100 ppm, ou 50 ppm, ou 25 ppm, ou 10 ppm, ou 5 ppm, de F-1234ze.

De préférence, ces teneurs sont obtenues à l'issue de la fluoration, sans (ou avant toute) étape de purification du flux de produit.

### EXEMPLES

Les exemples ci-dessous illustrent de façon comparative la réaction de fluoration de compositions à base de F-240db pour obtenir du F-1234yf.

Le catalyseur utilisé (50 mL) est un catalyseur massique à base d'oxyde de chrome.

L'activation comprend les étapes suivantes :
- Une étape de séchage à pression atmosphérique sous courant d'azote à une température d'environ 275°C pendant 120 heures.
- Une première étape d'activation à une température d'environ 275°C sous un mélange azote et acide fluorhydrique en réduisant progressivement l'azote pendant 41 heures. On observe ensuite un palier sous HF pur pendant 41 heures en augmentant la température jusqu'à 350°C.
- Une deuxième étape d'activation avec de l'air pendant 101 heures à 350°C avant de réaliser la réaction de fluoration.

### Exemple 1

Dans cet exemple, on procède à une réaction de fluoration à partir d'une composition contenant du F-240db à une pureté élevée (teneur en F-240db supérieure à 99,95 %).

Les conditions de la réaction sont les suivantes :
- rapport molaire HF / F-240db : 20 ;
- rapport molaire oxygène / F-240db : 0,2 ;
- temps de contact : 10 s ;
- pression : pression atmosphérique ;
- température : 350°C.

Après 125 heures dans ces conditions, le flux gazeux sortant du réacteur est analysé, après lavage, par chromatographie en phase gazeuse. La conversion du F-240db est de 100 %. L'analyse du flux est reportée dans le tableau ci-dessous (valeurs en % molaire) :

| **Produit détecté** | **Concentration** |
|---|---|
| CO | 0,91 |
| CO₂ | 1,50 |
| F-143a | 0,23 |
| F-1234yf | 8,31 |
| F-245cb | 1,84 |
| F-1233xf | 85,98 |
| F-1223xd | 1,23 |
| Autres | traces |

A l'issue de cette étape préliminaire de réaction, les produits sont séparés par distillation sur une colonne à garnissage de type Sulzer. Le F-1234yf obtenu est très pur (pureté supérieure à 99,95 %).

### Exemple 2

Dans cet exemple, on procède à une réaction de fluoration à partir d'une composition contenant 99,43 % de F-240db et 0,57 % de F-250fb.

Les conditions de la réaction sont les suivantes :
- rapport molaire HF / organiques chlorés : 20 ;
- rapport molaire oxygène / organiques chlorés : 0,2 ;
- temps de contact : 10 s ;
- pression : pression atmosphérique ;
- température : 350°C.

Après 51 heures dans ces conditions, le flux gazeux sortant du réacteur est analysé, après lavage, par chromatographie en phase gazeuse. La conversion du F-240db et du F-250fb est de 100 %. L'analyse du flux est reportée dans le tableau ci-dessous (valeurs en % molaire) :

| **Produit détecté** | **Concentration** |
|---|---|
| CO | 1,63 |
| CO₂ | 0,82 |
| F-143a | 0,22 |
| F-1243zf | 0,54 |
| F-1234yf | 12,67 |
| F-245cb | 3,02 |
| F-1242zf | traces |
| F-1233xf | 80,49 |
| F-253fb | traces |
| F-1223xd | 0,61 |
| Autres | traces |

A l'issue de cette étape préliminaire de réaction, les produits sont séparés par distillation sur une colonne à garnissage de type Sulzer. Les résultats obtenus montrent que le F-1243zf et le F-1234yf ne se séparent pas par distillation.

Industriellement, l'utilisation d'une telle matière première conduirait à l'obtention d'un flux de F-1234yf contenant plus de 500 ppm de F-1243zf.

### Exemple 3

Dans cet exemple, on procède à une réaction de fluoration d'une composition contenant 99,62 % de F-240db et 0,38 % de F-230fa.

Les conditions de la réaction sont les mêmes que dans l'exemple 2.

Après 62 heures dans ces conditions, le flux gazeux sortant du réacteur est analysé, après lavage, par chromatographie en phase gazeuse. La conversion du F-240db et du F-230fa est de 100 %. L'analyse du flux est reportée dans le tableau ci-dessous (valeurs en % molaire) :

| **Produit détecté** | **Concentration** |
|---|---|
| CO | 2,03 |
| CO₂ | 1,32 |
| F-143a | 0,19 |
| F-1225zc | 0,02 |
| F-1234yf | 11,04 |
| F-245cb | 2,41 |
| F-236fa | 0,18 |
| F-1224 | 0,04 |
| F-1233xf | 81,51 |
| F-226da | traces |
| F-235fa | 0,01 |
| F-1214 | traces |
| F-1223xd | 1,24 |
| F-1213xa | 0,01 |
| Inconnus | traces |

A l'issue de cette étape préliminaire de réaction, les produits sont séparés par distillation sur une colonne à garnissage de type Sulzer. Les résultats obtenus montrent que le F-1225zc et le F-1234yf se séparent mal par distillation.

Industriellement, l'utilisation d'une telle matière première conduirait à l'obtention d'un flux de F-1234yf contenant plus de 500 ppm de F-1225zc.

## Revendications

1. Composition comprenant au moins 99 % en poids de 1,1,1,2,3-pentachloropropane, et comprenant au moins un composé choisi parmi une liste de composés supplémentaires consistant en les trichloropropanes, les tétrachloropropanes, les pentachloropropanes différents du 1,1,1,2,3-pentachloropropane, les hexachloropropanes, les heptachloropropanes, les dichloropropènes, les trichloropropènes, les tétrachloropropènes, les pentachloropropènes et l'hexachloropropène, ledit composé étant présent dans la composition en une teneur pondérale inférieure ou égale à 500 ppm.

2. Composition selon la revendication 1, dans laquelle ledit composé est présent dans la composition en une teneur pondérale inférieure ou égale à 250 ppm ; de préférence inférieure ou égale à 150 ppm ; plus particulièrement inférieure ou égale à 100 ppm ; plus particulièrement inférieure ou égale à 50 ppm ; et idéalement inférieure ou égale à 10 ppm.

3. Composition selon la revendication 1 ou 2, comprenant une pluralité de composés choisis parmi ladite liste de composés supplémentaires, chacun des composés de ladite pluralité de composés étant présent dans la composition en une teneur pondérale inférieure ou égale à 500 ppm ; de préférence inférieure ou égale à 250 ppm ; de préférence inférieure ou égale à 150 ppm; plus particulièrement inférieure ou égale à 100 ppm ; plus particulièrement inférieure ou égale à 50 ppm ; et idéalement inférieure ou égale à 10 ppm.

4. Composition selon l'une des revendications 1 à 3, comprenant une pluralité de composés choisis parmi ladite liste de composés supplémentaires, la teneur pondérale totale de l'ensemble des composés de ladite liste étant inférieure ou égale à 1000 ppm ; de préférence inférieure ou égale à 500 ppm ; de préférence inférieure ou égale à 250 ppm ; de préférence inférieure ou égale à 150 ppm ; plus particulièrement inférieure ou égale à 100 ppm ; plus particulièrement inférieure ou égale à 50 ppm ; et idéalement inférieure ou égale à 10 ppm.

5. Composition selon l'une des revendications 1 à 4, qui comprend au moins 99,5 % en poids, et de préférence au moins 99,8 % en poids, et de manière plus particulièrement préférée au moins 99,9 % en poids, de 1,1,1,2,3-pentachloropropane.

6. Composition selon l'une des revendications 1 à 5, comprenant au moins un composé choisi parmi le groupe constitué du 1,1,1,3-tétrachloropropane, du 3,3,3-trichloropropène et du 1,1,3-trichloropropène, et dans laquelle la teneur pondérale de chacun de ces composés dans la composition est inférieure ou égale à 100 ppm, de préférence inférieure ou égale à 50 ppm ; et dans laquelle, optionnellement, la teneur pondérale totale des composés de ce groupe dans la composition est inférieure ou égale à 100 ppm, de préférence inférieure ou égale à 50 ppm.

7. Composition selon l'une des revendications 1 à 6, comprenant au moins un composé choisi parmi les trichloropropènes et les tétrachloropropanes, la teneur pondérale de chacun de ces composés dans la composition étant inférieure ou égale à 250 ppm, de préférence inférieure ou égale à 150 ppm ; et dans laquelle, optionnellement, la teneur pondérale totale des trichloropropènes et tétrachloropropanes dans la composition est inférieure ou égale à 250 ppm, de préférence inférieure ou égale à 150 ppm.

8. Composition selon l'une des revendications 1 à 7, comprenant au moins un composé choisi parmi les pentachloropropènes et les hexachloropropanes, la teneur pondérale de chacun de ces composés dans la composition étant inférieure ou égale à 50 ppm, de préférence inférieure ou égale à 10 ppm; et dans laquelle, optionnellement, la teneur pondérale totale des pentachloropropènes et hexachloropropanes dans la composition est inférieure ou égale à 50 ppm, de préférence inférieure ou égale à 10 ppm.

9. Composition selon l'une des revendications 1 à 8, comprenant au moins un composé choisi parmi l'hexachloropropène et les heptachloropropanes, la teneur pondérale de chacun de ces composés dans la composition étant inférieure ou égale à 50 ppm, de préférence inférieure ou égale à 10 ppm; et dans laquelle, optionnellement, la teneur pondérale totale de l'hexachloropropène et des heptachloropropanes dans la composition est inférieure ou égale à 50 ppm, de préférence inférieure ou égale à 10 ppm.

10. Composition selon l'une des revendications 1 à 9, comprenant au moins un composé choisi parmi les dichloropropènes et les trichloropropanes, la teneur pondérale de chacun de ces composés dans la composition étant inférieure ou égale à 50 ppm, de préférence inférieure ou égale à 10 ppm; et dans laquelle, optionnellement, la teneur pondérale totale des dichloropropènes et trichloropropanes dans la composition est inférieure ou égale à 50 ppm, de préférence inférieure ou égale à 10 ppm.

11. Composition selon l'une des revendications 1 à 10, comprenant au moins un composé choisi parmi le groupe constitué du 1,1,3,3-tétrachloropropène, du 1,3,3,3-tétrachloropropène, du 1,1,1,3,3-pentachloropropane et du 1,1,2,3,3-pentachloropropane, et dans laquelle la teneur pondérale de chacun de ces composés dans la composition est inférieure ou égale à 500 ppm, de préférence inférieure ou égale à 300 ppm ; et dans laquelle, optionnellement, la teneur pondérale totale des composés de ce groupe dans la composition est inférieure ou égale à 500 ppm, de préférence inférieure ou égale à 300 ppm.

12. Procédé de production de 2,3,3,3-tétrafluoropropène, comprenant :
- la fourniture d'une composition selon l'une des revendications 1 à 11 ;
- la réaction de cette composition avec de l'acide fluorhydrique en phase gazeuse.

13. Procédé selon la revendication 12, comprenant une unique étape de fluoration catalytique.

14. Procédé selon la revendication 12, comprenant deux étapes successives de fluoration catalytique, à savoir :
- la réaction de la composition avec de l'acide fluorhydrique en phase gazeuse, pour fabriquer un produit intermédiaire ;
- optionnellement, une purification du produit intermédiaire ; puis
- la réaction du produit intermédiaire avec de l'acide fluorhydrique en phase gazeuse, pour fabriquer le 2,3,3,3-tétrafluoropropène ;
le produit intermédiaire étant de préférence le 2-chloro-3,3,3-trifluoropropène.

## Patentansprüche

1. Zusammensetzung, umfassend mindestens 99 Gew.-% 1,1,1,2,3-Pentachlorpropan und mindestens eine Verbindung aus einer Liste zusätzlicher Verbindungen bestehend aus Trichlorpropanen, Tetrachlorpropanen, Pentachlorpropanen, die von 1,1,1,2,3-Pentachlorpropan verschieden sind, Hexachlorpropanen, Heptachlorpropanen, Dichlorpropenen, Trichlorpropenen, Tetrachlorpropenen, Pentachlorpropenen und Hexachlorpropen, wobei die Verbindung in der Zusammensetzung in einem Gewichtsgehalt kleiner oder gleich 500 ppm vorliegt.

2. Zusammensetzung nach Anspruch 1, wobei die Verbindung in der Zusammensetzung in einem Gewichtsgehalt kleiner oder gleich 250 ppm, vorzugsweise kleiner oder gleich 150 ppm, spezieller kleiner oder gleich 100 ppm, spezieller kleiner oder gleich 50 ppm und idealerweise kleiner oder gleich 10 ppm vorliegt.

3. Zusammensetzung nach Anspruch 1 oder 2, umfassend mehrere Verbindungen aus der Liste zusätzlicher Verbindungen, wobei jede der Verbindungen der mehreren Verbindungen in der Zusammensetzung in einem Gewichtsgehalt kleiner oder gleich 500 ppm, vorzugsweise kleiner oder gleich 250 ppm, vorzugsweise kleiner oder gleich 150 ppm, spezieller kleiner oder gleich 100 ppm, spezieller kleiner oder gleich 50 ppm und idealerweise kleiner oder gleich 10 ppm vorliegt.

4. Zusammensetzung nach einem der Ansprüche 1 bis 3, umfassend mehrere Verbindungen aus der Liste zusätzlicher Verbindungen, wobei der gesamte Gewichtsgehalt aller Zusammensetzungen der Liste kleiner oder gleich 1000 ppm, vorzugsweise kleiner oder gleich 500 ppm, vorzugsweise kleiner oder gleich 250 ppm, vorzugsweise kleiner oder gleich 150 ppm, spezieller kleiner oder gleich 100 ppm, spezieller kleiner oder gleich 50 ppm und idealerweise kleiner oder gleich 10 ppm ist.

5. Zusammensetzung nach einem der Ansprüche 1 bis 4, die mindestens 99,5 Gew.-% und vorzugsweise mindestens 99,8 Gew.-% und spezieller bevorzugt mindestens 99,9 Gew.-% 1,1,1,2,3-Pentachlorpropan umfasst.

6. Zusammensetzung nach einem der Ansprüche 1 bis 5, umfassend mindestens eine Verbindung aus der Gruppe bestehend aus 1,1,1,3-Tetrachlorpropan, 3,3,3-Trichlorpropen und 1,1,3-Trichlorpropen, wobei der Gewichtsgehalt jeder dieser Verbindungen in der Zusammensetzung kleiner oder gleich 100 ppm, vorzugsweise kleiner oder gleich 50 ppm, ist und wobei gegebenenfalls der gesamte Gewichtsgehalt der Verbindungen dieser Gruppe in der Zusammensetzung kleiner oder gleich 100 ppm, vorzugsweise kleiner oder gleich 50 ppm, ist.

7. Zusammensetzung nach einem der Ansprüche 1 bis 6, umfassend mindestens eine Verbindung, die aus Trichlorpropenen und Tetrachlorpropanen ausgewählt ist, wobei der Gewichtsgehalt jeder dieser Verbindungen in der Zusammensetzung kleiner oder gleich 250 ppm, vorzugsweise kleiner oder gleich 150 ppm, ist und wobei gegebenenfalls der gesamte Gewichtsgehalt der Trichlorpropene und Tetrachlorpropane in der Zusammensetzung kleiner oder gleich 250 ppm, vorzugsweise kleiner oder gleich 150 ppm, ist.

8. Zusammensetzung nach einem der Ansprüche 1 bis 7, umfassend mindestens eine Verbindung, die aus Pentachlorpropenen und Hexachlorpropanen ausgewählt ist, wobei der Gewichtsgehalt jeder dieser Verbindungen in der Zusammensetzung kleiner oder gleich 50 ppm, vorzugsweise kleiner oder gleich 10 ppm, ist und wobei gegebenenfalls der gesamte Gewichtsgehalt der Pentachlorpropene und Hexachlorpropane in der Zusammensetzung kleiner oder gleich 50 ppm, vorzugsweise kleiner oder gleich 10 ppm, ist.

9. Zusammensetzung nach einem der Ansprüche 1 bis 8, umfassend mindestens eine Verbindung, die aus Hexachlorpropen und Heptachlorpropanen ausgewählt ist, wobei der Gewichtsgehalt jeder dieser Verbindungen in der Zusammensetzung kleiner oder gleich 50 ppm, vorzugsweise kleiner oder gleich 10 ppm, ist und wobei gegebenenfalls der gesamte Gewichtsgehalt des Hexachlorpropens und der Heptachlorpropane in der Zusammensetzung kleiner oder gleich 50 ppm, vorzugsweise kleiner oder gleich 10 ppm, ist.

10. Zusammensetzung nach einem der Ansprüche 1 bis 9, umfassend mindestens eine Verbindung, die aus Dichlorpropenen und Trichlorpropanen ausgewählt ist, wobei der Gewichtsgehalt jeder dieser Verbindungen in der Zusammensetzung kleiner oder gleich 50 ppm, vorzugsweise kleiner oder gleich 10 ppm, ist und wobei gegebenenfalls der gesamte Gewichtsgehalt der Dichlorpropene und Trichlorpropane in der Zusammensetzung kleiner oder gleich 50 ppm, vorzugsweise kleiner oder gleich 10 ppm, ist.

11. Zusammensetzung nach einem der Ansprüche 1 bis 10, umfassend mindestens eine Verbindung, die aus der Gruppe bestehend aus 1,1,3,3-Tetrachlorpropen, 1,3,3,3-Tetrachlorpropen, 1,1,1,3,3-Pentachlorpropan und 1,1,2,3,3-Pentachlorpropan ausgewählt ist, wobei der Gewichtsgehalt jeder dieser Verbindungen in der Zusammensetzung kleiner oder gleich 500 ppm, vorzugsweise kleiner oder gleich 300 ppm, ist und wobei gegebenenfalls der gesamte Gewichtsgehalt der Verbindungen dieser Gruppe in der Zusammensetzung kleiner oder gleich 500 ppm, vorzugsweise kleiner oder gleich 300 ppm, ist.

12. Verfahren zur Herstellung von 2,3,3,3-Tetrafluorpropen, umfassend:
- Bereitstellung einer Zusammensetzung nach einem der Ansprüche 1 bis 11;
- Umsetzung dieser Zusammensetzung mit Fluorwasserstoffsäure in der Gasphase.

13. Verfahren nach Anspruch 12, das einen einzigen Schritt der katalytischen Fluorierung umfasst.

14. Verfahren nach Anspruch 12, das zwei aufeinanderfolgende Schritte der katalytischen Fluorierung umfasst, nämlich:
- Umsetzung der Zusammensetzung mit Fluorwasserstoffsäure in der Gasphase zur Herstellung eines Zwischenprodukts;
- gegebenenfalls eine Reinigung des Zwischenprodukts; dann
- Umsetzung des Zwischenprodukts mit Fluorwasserstoffsäure in der Gasphase zur Herstellung von 2,3,3,3-Tetrafluorpropen;
wobei es sich bei dem Zwischenprodukt vorzugsweise um 2-Chlor-3,3,3-trifluorpropen handelt.

## Claims

1. Composition comprising at least 99% by weight of 1,1,1,2,3-pentachloropropane, and comprising at least one compound chosen from a list of additional compounds consisting of trichloropropanes, tetrachloropropanes, pentachloropropanes other than 1,1,1,2,3-pentachloropropane, hexachloropropanes, heptachloropropanes, dichloropropenes, trichloropropenes, tetrachloropropenes, pentachloropropenes and hexachloropropene, said compound being present in the composition in a weight content of less than or equal to 500 ppm.

2. Composition according to Claim 1, in which said compound is present in the composition in a weight content of less than or equal to 250 ppm; preferably less than or equal to 150 ppm; more particularly less than or equal to 100 ppm; more particularly less than or equal to 50 ppm; and ideally less than or equal to 10 ppm.

3. Composition according to Claim 1 or 2, comprising a plurality of compounds chosen from said list of additional compounds, each of the compounds of said plurality of compounds being present in the composition in a weight content of less than or equal to 500 ppm; preferably less than or equal to 250 ppm; preferably less than or equal to 150 ppm; more particularly less than or equal to 100 ppm; more particularly less than or equal to 50 ppm; and ideally less than or equal to 10 ppm.

4. Composition according to one of Claims 1 to 3, comprising a plurality of compounds chosen from said list of additional compounds, the total weight content of all of the compounds of said list being less than or equal to 1000 ppm; preferably less than or equal to 500 ppm; preferably less than or equal to 250 ppm; preferably less than or equal to 150 ppm; more particularly less than or equal to 100 ppm; more particularly less than or equal to 50 ppm; and ideally less than or equal to 10 ppm.

5. Composition according to one of Claims 1 to 4, which comprises at least 99.5% by weight, preferably at least 99.8% by weight, and more particularly preferably at least 99.9% by weight, of 1,1,1,2,3-pentachloropropane.

6. Composition according to one of Claims 1 to 5, comprising at least one compound chosen from the group consisting of 1,1,1,3-tetrachloropropane, 3,3,3-trichloropropene and 1,1,3-trichloropropene, and in which the weight content of each of these compounds in the composition is less than or equal to 100 ppm, preferably less than or equal to 50 ppm; and in which, optionally, the total weight content of the compounds of this group in the composition is less than or equal to 100 ppm, preferably less than or equal to 50 ppm.

7. Composition according to one of Claims 1 to 6, comprising at least one compound chosen from trichloropropenes and tetrachloropropanes, the weight content of each of these compounds in the composition being less than or equal to 250 ppm, preferably less than or equal to 150 ppm; and in which, optionally, the total weight content of the trichloropropenes and tetrachloropropanes in the composition is less than or equal to 250 ppm, preferably less than or equal to 150 ppm.

8. Composition according to one of Claims 1 to 7, comprising at least one compound chosen from pentachloropropenes and hexachloropropanes, the weight content of each of these compounds in the composition being less than or equal to 50 ppm, preferably less than or equal to 10 ppm; and in which, optionally, the total weight content of the pentachloropropenes and hexachloropropanes in the composition is less than or equal to 50 ppm, preferably less than or equal to 10 ppm.

9. Composition according to one of Claims 1 to 8, comprising at least one compound chosen from hexachloropropene and heptachloropropanes, the weight content of each of these compounds in the composition being less than or equal to 50 ppm, preferably less than or equal to 10 ppm; and in which, optionally, the total weight content of hexachloropropene and of heptachloropropanes in the composition is less than or equal to 50 ppm, preferably less than or equal to 10 ppm.

10. Composition according to one of Claims 1 to 9, comprising at least one compound chosen from dichloropropenes and trichloropropanes, the weight content of each of these compounds in the composition being less than or equal to 50 ppm, preferably less than or equal to 10 ppm; and in which, optionally, the total weight content of the dichloropropenes and trichloropropanes in the composition is less than or equal to 50 ppm, preferably less than or equal to 10 ppm.

11. Composition according to one of Claims 1 to 10, comprising at least one compound chosen from the group consisting of 1,1,3,3-tetrachloropropene, 1,3,3,3-tetrachloropropene, 1,1,1,3,3-pentachloropropane and 1,1,2,3,3-pentachloropropane, and in which the weight content of each of these compounds in the composition is less than or equal to 500 ppm, preferably less than or equal to 300 ppm; and in which, optionally, the total weight content of the compounds of this group in the composition is less than or equal to 500 ppm, preferably less than or equal to 300 ppm.

12. Process for producing 2,3,3,3-tetrafluoropropene, comprising:
- the provision of a composition according to one of Claims 1 to 11;
- the reaction of this composition with hydrofluoric acid in the gas phase.

13. Process according to Claim 12, comprising a single step of catalytic fluorination.

14. Process according to Claim 12, comprising two successive steps of catalytic fluorination, namely:
- the reaction of the composition with hydrofluoric acid in the gas phase, to manufacture an intermediate product;
- optionally, purification of the intermediate product; and then
- the reaction of the intermediate product with hydrofluoric acid in the gas phase, to manufacture 2,3,3,3-tetrafluoropropene;
the intermediate product preferably being 2-chloro-3,3,3-trifluoropropene.
